# EUROPEAN PATENT APPLICATION

(11) **EP 4 321 613 A1**
(43) Date of publication of application: **14.02.2024**
(21) Application number: 22798960.5
(22) Date of filing: 06.05.2022
(51) Int. Cl.: C12N 5/0783, A61K 35/17, A61P 31/00, A61P 35/00, A61P 37/02, C12N 5/0781

(54) **METHOD FOR PRODUCING MEMORY T CELLS**

(30) Priority: 07.05.2021 JP 2021079296
(71) Applicant: Tokumoto, Yasuhito, Tokyo 179-0075 (JP)
(72) Inventor: TOKUMOTO, Yasuhito, Tokyo 179-0075 (JP); MIMURA, Toshihide, Tokyo 167-0052 (JP); ARAKI, Yasuto, Sakado-shi, Saitama 350-0231 (JP); OHSHIMA, Susumu, Tagata-gun, Shizuoka 419-0107 (JP)
(74) Representative: Nederlandsch Octrooibureau
(86) International application number: PCT/JP2022/019563
(87) International publication number: WO 2022/234856

(57) **Abstract**

The present invention provides a method of manufacturing memory T cells from naive T cells and a composition containing the memory T cells manufactured by the method. The present invention is a manufacturing method of memory T cells including an activation step of activating naive T cells with a differentiation-inducing factor and a memory-formation step of culturing the activated T cells in the absence of a differentiation-inducing factor after the activation step to manufacture memory T cells, in which the activated T cells are cultured in a hypoxic environment in the memory-formation step. The present invention is also a memory T cell-containing composition in which a total number of living cells of stem cell memory T cells, central memory T cells, and effector memory T cells is equal to or more than 20% of a total number of living cells contained in the composition.

## Description

### [Technical Field]

The present invention relates to a method of conveniently manufacturing memory T cells from naive T cells.

Priority is claimed on Japanese Patent Application No. 2021-079296 filed May 7, 2021, the content of which is incorporated herein by reference.

### [Background Art]

Both T cells and B cells are cells that play an important role in immunity. T cells are lymphocytes that are differentiated and matured from progenitor cells through thymic selection after being produced in the bone marrow. T cells present in the periphery are mainly classified into CD4⁺ T cells and CD8⁺ T cells. CD4⁺ T cells are cells that express CD4 as a cell surface antigen, regulate the functions of other T cells or B cells in response to antigenic stimulation, and are called helper T cells. CD4⁺ T cells are classified into subgroups such as Th1 cells, Th2 cells, and Th17 cells, according to the type of cytokine that the CD4⁺ T cells secrete. CD8⁺ T cells are cells that express CD8 as a cell surface antigen and are responsible for cellular immunity in which the CD8⁺ T cells kill virus-infected cells and the like, and activated effector cells are also called killer T cells. On the other hand, B cells are lymphocytes differentiated from hematopoietic cells in the bone marrow, stimulated by helper T cells, and responsible for humoral immunity in which the B cells are differentiated into plasma cells by the stimulation of helper T cells and produce antibodies.

When recognizing antigens presented by antigen-presenting cells, naive T cells are activated and differentiate into effector cells. When activated, the naive CD4⁺ T cells differentiate into helper CD4⁺ Th1 cells, helper CD4⁺ Th2 cells, or the like. When activated, the naive CD8⁺ T cells differentiate into killer T cells. These effector cells work together to eliminate pathogens and the like. These effector cells have a short life and die within about several days to weeks unless continuously exposed to antigens. However, some of the effector cells become memory cells and survive for a long period of time. These memory cells enable a rapid immune response upon reinfection with the same pathogen.

In recent years, cancer treatments utilizing the immune system have also been carried out. In these cancer immunotherapies, in order to obtain a higher therapeutic effect, it is important for the effector cells to be formed into memory cells and survive for a long period of time. For example, vaccine therapies for cancer are therapies that cause antigen-presenting cells (dendritic cells) to present cancer cell surface antigens and stimulate T cells to activate the immune system and eliminate cancer. One of the vaccine therapies for cancer is a DC vaccine therapy in which antigen-presenting cells collected from a patient are caused to engulf cancer cells or cancer cell surface antigens to manufacture antigen-presenting cells presenting cancer cell surface antigens, and the antigen-presenting cells are allowed to expand, are activated, and then returned back into the patient's body. In this method, it is preferable that memory cells be included in various activated lymphocytes returned back into the body together with the activated cancer cell surface antigen-presenting cells returned back into the patient's body. A CAR-T cell therapy is a therapy in which T cells collected from a patient are modified by a gene recombination technique to express a chimeric antigen receptor (CAR) that recognizes cancer cell surface antigens, are allowed to expand, and then returned back into the patient's body. The modified T cells (CAR-T cells) returned back into the body recognize and attack cancer cells, and in this way, cancer cells are eliminated. It is preferable that memory cells be included in the CAR-T cell population returned back into the body. In these therapies, in a case where lymphocytes to be returned back into the patient's body include memory cells, long-term survival of these memory cells makes it possible to eliminate cancer for a long period of time and is effective for preventing recurrence of cancer as well.

However, it is extremely difficult to form T cells into memory cells ex vivo. For example, in the DC vaccine therapy or CAR-T cell therapy, activated cancer cell surface antigen-presenting cells or CAR-T cells are cultured, and cells whose proliferative ability does not decline over a long period of time are treated as memory cells. In this method, although long-term expansion is possible, the cells cannot keep expanding and die in a case where the cells do not receive stimulation from antigens or cytokines once every several days or 1 to 2 weeks. However, even though there is no stimulation from cytokines or antigens, natural memory T cells can survive for a long period of time in a state where the cells have ceased expanding (Quiescence). Furthermore, the natural memory T cells are small spherical cells, whereas the memory T cells obtained ex vivo are blast cells having irregular shapes (Non-Patent Document 1).

### [Citation List]

### [Non-Patent Document]

[Non-Patent Document 1]
Kaartinen et al., Cytotherapy, 2017, vol.19, p.689-702.

### [Summary of Invention]

### [Technical Problem]

A main object of the present invention is to provide a method of manufacturing memory T cells from naive T cells, a composition containing memory T cells manufactured by the method, and the like.

### [Solution to Problem]

As a result of intensive studies, the inventors of the present invention have found that activating naive T cells with a differentiation-inducing factor and then culturing the activated T cells in a differentiation-inducing factor-free medium in a hypoxic environment makes it possible to manufacture memory T cells, and that treating naive B cells in the same way makes it possible to obtain cells viable independently of a differentiation-inducing factor. Based on the finding, the inventors have accomplished the present invention.

That is, a manufacturing method of memory T cells, a memory T cell-containing composition, a pharmaceutical composition, and a manufacturing method of cells viable independently of a differentiation-inducing factor according to the present invention are [1] to [12] described below.
[1] A manufacturing method of memory T cells, including an activation step of activating naive T cells with a differentiation-inducing factor, and
   a memory-formation step of culturing the activated T cells in the absence of a differentiation-inducing factor after the activation step to manufacture memory T cells,
   in which the activated T cells are cultured in a hypoxic environment in the memory-formation step.
[2] The manufacturing method of memory T cells described in [1], in which an oxygen concentration of the hypoxic environment is 0.5% to 5.0% by volume.
[3] A manufacturing method of memory T cells, including an activation step of activating naive T cells with a differentiation-inducing factor, and
   a memory-formation step of culturing the activated T cells in the absence of a differentiation-inducing factor after the activation step to manufacture memory T cells,
   in which the naive T cells are cells having been subjected to a stress treatment, or a stress treatment is performed on the activated T cells before the memory-formation step.
[4] The manufacturing method of memory T cells described in [3], in which the naive T cells are cells having been kept at 0°C to 10°C for 1 hour or longer.
[5] The manufacturing method of memory T cells described in [3], in which the naive T cells are cells collected from a human aged 50 or over.
[6] The manufacturing method of memory T cells described in [3], in which the memory-formation step is performed after the activated T cells are frozen and thawed.
[7] The manufacturing method of memory T cells described in any one of [1] to [6], in which the naive T cells are naive CD4⁺ T cells or naive CD8⁺ T cells.
[8] The manufacturing method of memory T cells described in [7], in which the differentiation-inducing factor is a differentiation-inducing factor for inducing differentiation of naive CD4⁺ T cells into CD4⁺ Th1 cells, for inducing differentiation of naive CD4⁺ T cells into CD4⁺ Th2 cells, or for inducing differentiation of naive CD8⁺ T cells into killer T cells.
[9] A memory T cell-containing composition, in which a total number of living cells of stem cell memory T cells, central memory T cells, and effector memory T cells is equal to or more than 20% of a total number of living cells contained in the composition.
[10] A pharmaceutical composition containing the memory T cell-containing composition described in [9] as an active ingredient.
[11] A manufacturing method of cells viable for a long period of time independently of a differentiation-inducing factor, the manufacturing method including an activation step of activating naive B cells with a differentiation-inducing factor, and
   a long-term viability acquisition step of culturing the activated B cells in the absence of a differentiation-inducing factor after the activation step to manufacture cells viable independently of a differentiation-inducing factor,
   in which the activated B cells are cultured in a hypoxic environment in the long-term viability acquisition step.
[12] The manufacturing method of cells viable for a long period of time independently of a differentiation-inducing factor described in [11], in which the cells viable for a long period of time independently of a differentiation-inducing factor are one or more kinds of cells selected from plasma cells, germinal center B cells, and transitional B cells.

### [Advantageous Effects of Invention]

According to the present invention, memory T cells can be very efficiently and conveniently manufactured from activated T cells.

In addition, according to the present invention, B cells that are viable independently of a differentiation-inducing factor can be very efficiently and conveniently manufactured from activated B cells.

### [Brief Description of Drawings]

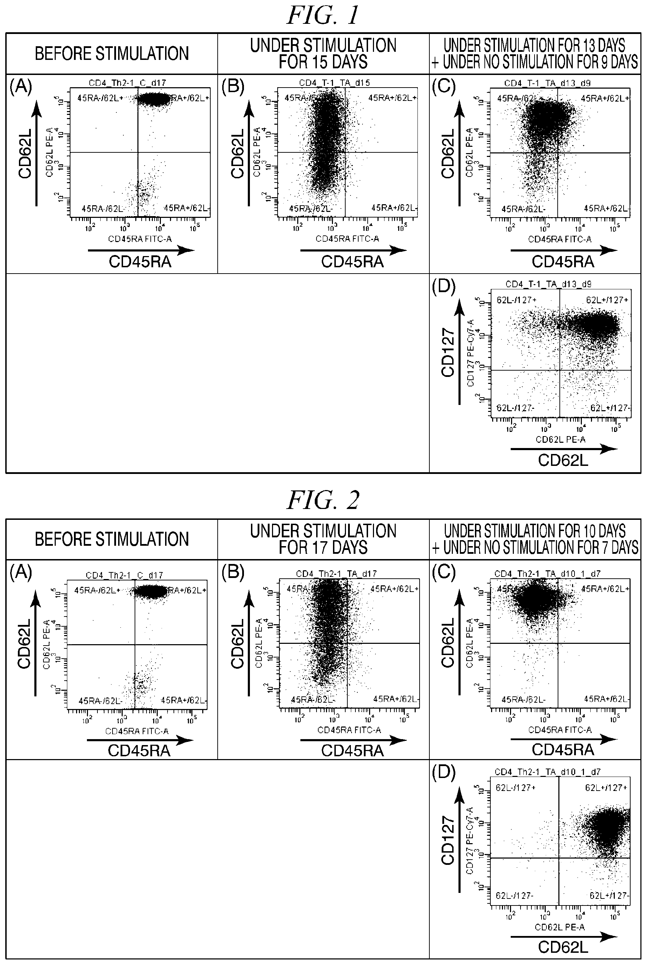

FIG. 1 is a view showing the results of FACS analysis on cells obtained by culturing naive CD4⁺ T cells in a Th1 medium and then in a differentiation-inducing factor deficient medium in a hypoxic environment in Example 1. FIG. 1(A) is a dot plot of naive CD4⁺ T cells (cells before stimulation) before differentiation induction, FIG. 1(B) is a dot plot of cells cultured in a Th1 medium for 15 days (cells under stimulation for 15 days), and FIGS. 1(C) and 1(D) are dot plots of cells cultured for 13 days in a Th1 medium and then cultured in a differentiation-inducing factor deficient medium for 9 days in a hypoxic environment (cells under stimulation for 13 days + cells under no stimulation for 9 days).
FIG. 2 is a view showing the results of FACS analysis on cells obtained by culturing naive CD4⁺ T cells in a Th2 medium and then in a differentiation-inducing factor deficient medium in a hypoxic environment in Example 1. FIG. 2(A) is a dot plot of naive CD4⁺ T cells (cells before stimulation) before differentiation induction, FIG. 2(B) is a dot plot of cells cultured in a Th2 medium for 17 days (cells under stimulation for 17 days), and FIGS. 2(C) and 2(D) are dot plots of cells cultured for 10 days in a Th2 medium and then cultured in a differentiation-inducing factor deficient medium for 7 days in a hypoxic environment (cells under stimulation for 10 days + cells under no stimulation for 7 days).

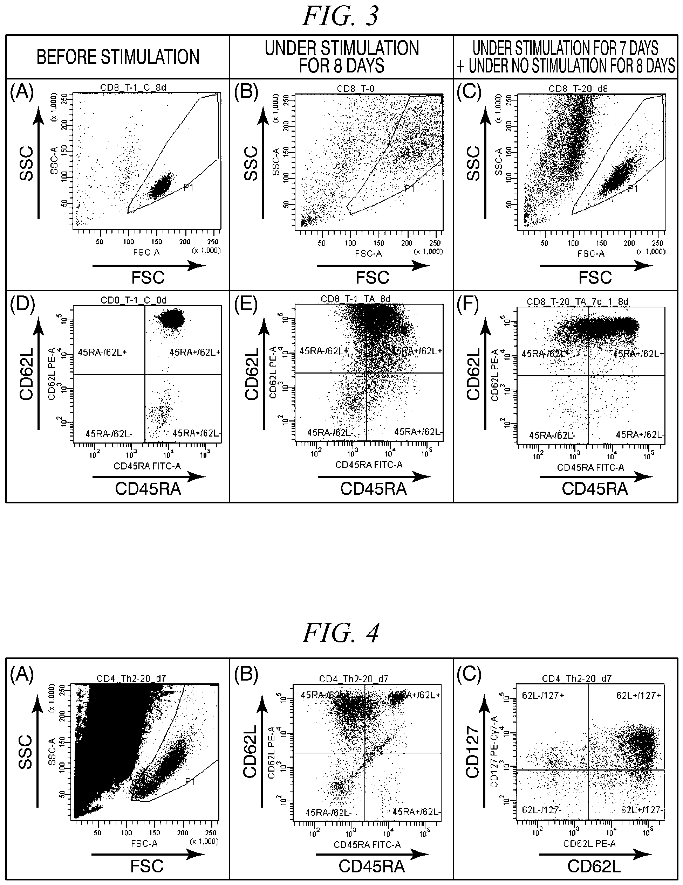

FIG. 3 is a view showing the results of FACS analysis on cells obtained by culturing naive CD8⁺ T cells in a CD8-TA medium and then in a differentiation-inducing factor deficient medium in a hypoxic environment in Example 2. FIGS. 3(A) and 3(D) are dot plots of naive CD8⁺ T cells (cells before stimulation) before differentiation induction, FIGS. 3(B) and 3(E) are dot plots of cells cultured in a CD8-TA medium for 8 days (cells under stimulation for 8 days), and FIGS. 3(C) and 3(F) are dot plots of cells cultured for 7 days in a CD8-TA medium and then cultured in a differentiation-inducing factor deficient medium for 8 days in a hypoxic environment (cells under stimulation for 7 days + cells under no stimulation for 8 days).
FIG. 4 is a dot plot showing the results of FACS analysis on cells obtained by culturing naive CD4⁺ T cells in a Th2 medium, followed by cryopreservation, and then culturing the cells in a differentiation-inducing factor deficient medium in a normal environment in Example 3.

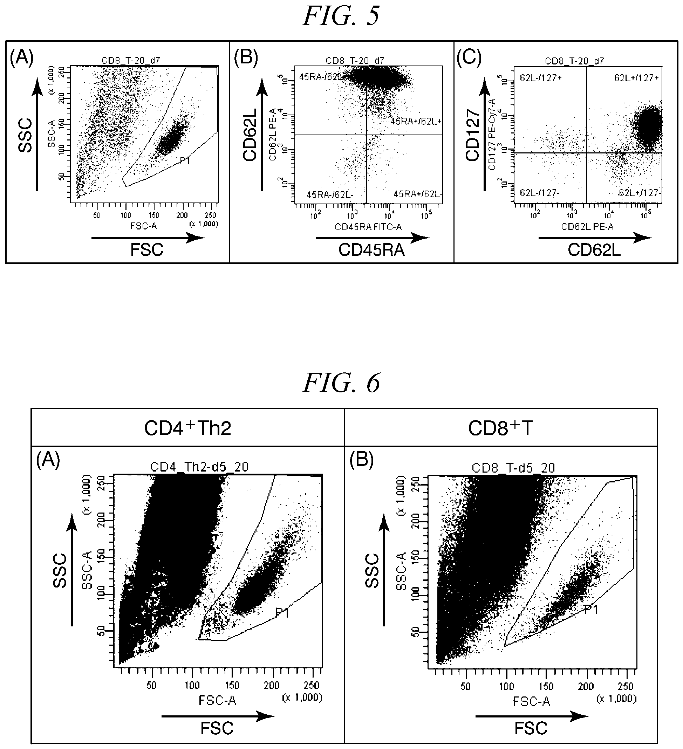

FIG. 5 is a dot plot showing the results of FACS analysis on cells obtained by culturing naive CD8⁺ T cells in a CD8-TA medium, followed by cryopreservation, and then culturing the cells in a differentiation-inducing factor deficient medium in a normal environment in Example 4.
FIG. 6 is a view showing the results of FACS analysis on cells obtained by inducing differentiation of cold-storaged naive CD4⁺ T cells (A) and naive CD8⁺ T cells (B) and then culturing the cells in a normal environment in Example 5.

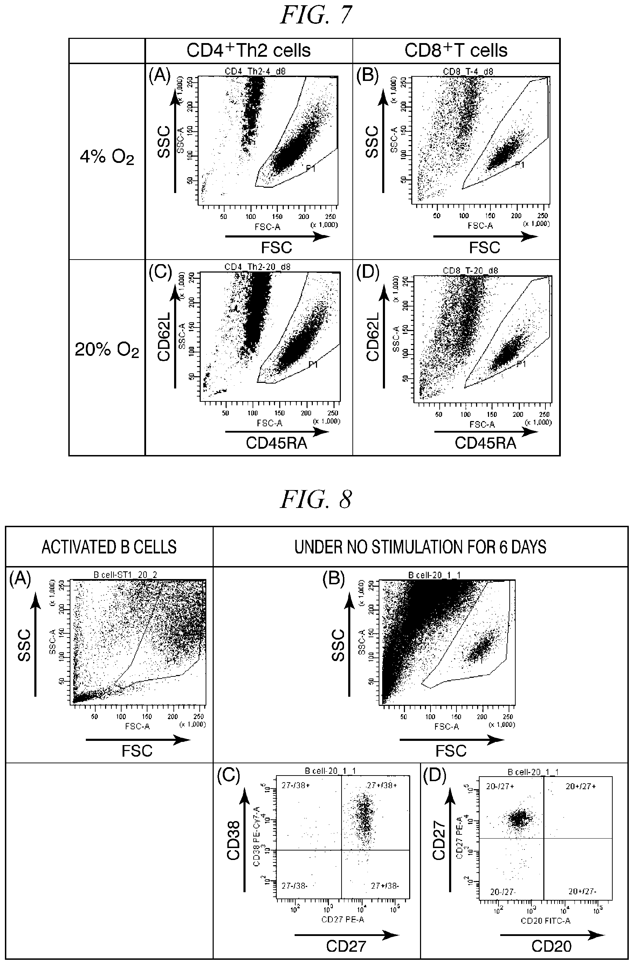

FIG. 7 is a view showing the FACS results on cells obtained by inducing differentiation of naive CD4⁺ T cells or naive CD8⁺ T cells collected from a 59-year-old human and then culturing the cells in a differentiation-inducing factor deficient medium in a hypoxic environment or a normal environment in Example 6.
FIG. 8 is a view showing the FACS results on cells (activated B cells) obtained by differentiation induction and activation of naive B cells in Example 7, and the FACS results on cells (cells under no stimulation for 6 days) obtained by culturing the activated B cells in a differentiation-inducing factor deficient medium in a hypoxic environment in Example 7.

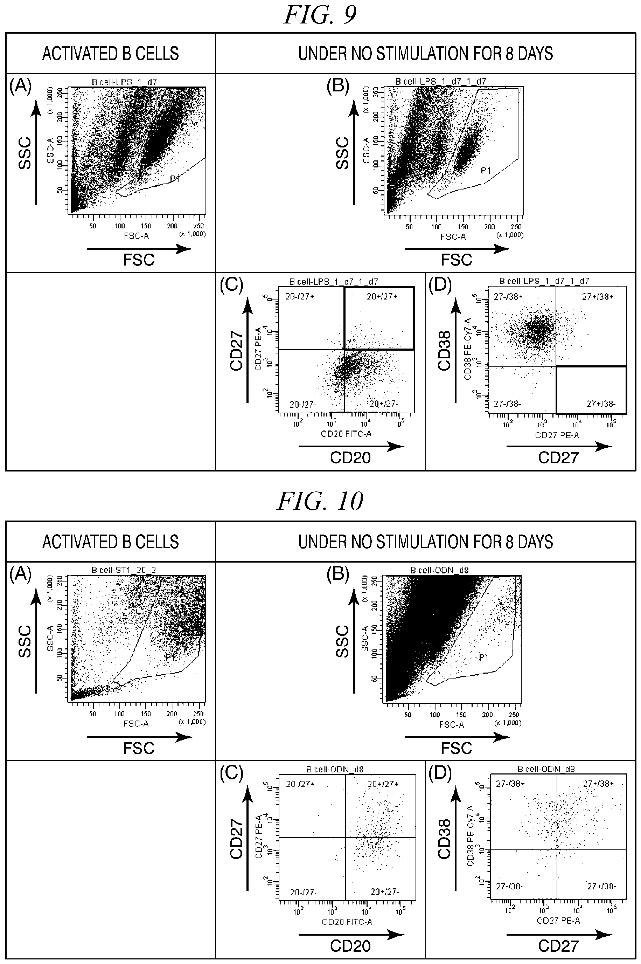

FIG. 9 is a view showing the FACS results on cells (activated B cells) obtained by differentiation induction and activation of naive B cells in Example 8, and the FACS results on cells (cells under no stimulation for 7 days) obtained by culturing the activated B cells in a differentiation-inducing factor deficient medium in a hypoxic environment in Example 8.
FIG. 10 is a view showing the FACS results on cells (activated B cells) obtained by differentiation induction and activation of naive B cells in Example 9, and the FACS results of cells (cells under no stimulation for 8 days) obtained by culturing the activated B cells in a differentiation-inducing factor deficient medium in a hypoxic environment in Example 9.

### [Description of Embodiments]

In the present invention and the present specification, "normal oxygen environment" refers to an environment in which the oxygen concentration is about the same as the atmospheric pressure, that is, about 19% to 21% by volume. In addition, "hypoxic environment" refers to an environment in which the oxygen concentration is 0.1% to 10% by volume.

In the present invention and present specification, "memory-formation" for T cells or B cells means that after ceasing expansion in an environment without stimulation from antigens or differentiation-inducing factors, the T cells or B cells acquire a trait of being able to survive for a long period of time while retaining an ability to differentiate into effector cells (undifferentiated and quiescent long-term viability), and the cell surface antigen-expressing pattern of these cells becomes the same as that of natural memory cells. Specifically, the undifferentiated and quiescent long-term viability means a trait of being able to survive for 7 days or more and preferably for 1 month or more in the absence of antigens or differentiation-inducing factors.

### <Manufacturing method of memory T cells>

The manufacturing method of memory T cells according to the present invention includes an activation step of activating naive T cells with a differentiation-inducing factor, and a memory-formation step of culturing the activated T cells in the absence of a differentiation-inducing factor after the activation step to manufacture memory T cells, in which the activated T cells are cultured in a hypoxic environment in the memory-formation step. The inventors of the present invention have found, for the first time, that adopting not only a normal oxygen environment but also a hypoxic environment in culturing T cells activated with a differentiation-inducing factor in the absence of a differentiation-inducing factor makes it possible to induce cellular quiescence (quiescent) and form memory.

The naive T cells used in the present invention may be either naive T cells collected from an animal or cells obtained by expanding naive T cells collected from an animal by primary culture or subculture. In addition, in the present invention, it is also possible to use naive T cells differentiated from embryonic stem (ES) cells and induced pluripotent stem (iPS) cells.

The naive T cells can be collected from a living body by a conventional method. For example, just as other lymphocytes, the naive T cells are in peripheral blood. Therefore, by fractionating peripheral blood mononuclear cells (PBMC) from the peripheral blood collected from animals and purifying naive T cells from PBMC, it is possible to collect naive T cells.

The PBMC fraction can be fractionated from the peripheral blood by a conventional method such as density gradient centrifugation using Ficoll (registered trademark). Furthermore, a commercially available PBMC preparation kit can also be used.

Cell surface antigens of the naive T cells are different from cell surface antigens of effector cells. For example, the naive T cells have CD45RA, CD62L, and CD127 among cell surface antigens. Therefore, by labeling PBMC with fluorescently labeled antibodies against these cell surface antigens and then using a fluorescence-activated cell sorter (FACS), it is possible to purify only a naive T cell population having CD45RA, CD62L, and CD127. Likewise, fractionating only a cell population having CD45RA, CD62L, CD127 and CD4 by FACS makes it possible to purify a naive CD4⁺ T cell population, and fractionating only a cell population having CD45RA, CD62L, CD127, and CD8 by FACS makes it possible to purify a naive CD8⁺ T cell population. The FACS device and the fluorescently labeled antibodies against various surface antigens to be used can be appropriately selected from those which are generally used, and commercially available products can also be used. In addition, instead of FACS, immunomagnetic beads composed of magnetic beads bound to antibodies against the aforementioned cell surface antigens can also be used to purify naive T cells from PBMC by using a magnet.

In the present invention, first, as the activation step, naive T cells are activated with a differentiation-inducing factor. Specifically, the naive T cells are cultured in a culture medium containing a differentiation-inducing factor. The differentiation-inducing factor to be used can be appropriately selected from basic factors such as an anti-human CD3 antibody or an anti-human CD3 antibody/anti-human CD28 antibody to provide an activation signal to a T cell receptor (TCR) and from cytokines necessary for inducing differentiation of naive T cells into effector cells. Examples of the cytokines include various growth factors such as interleukin (IL), interferon (IFN), and a transforming growth factor (TGF). For example, by stimulating naive CD4⁺ T cells with IL-12 and IFN-γ, it is possible to activate the cells to differentiate into CD4⁺ Th1 cells. By stimulating naive CD4⁺ T cells with IL-2, IL-4, IL-7, and thymic stromal lymphopoietin (TSLP), it is possible to activate the cells to differentiate into CD4⁺ Th2 cells. By stimulating naive CD4⁺ T cells with IL-21 and IL-6, it is possible to activate the cells to differentiate into CD4⁺ T_{FII} cells. By stimulating naive CD4⁺ T cells with TGFβ and IL-6, it is possible to activate the cells to differentiate into CD4⁺ Th17 cells. In addition, by stimulating naive CD8⁺ T cells with IL-2, it is possible to activate the cells to differentiate into CD8⁺ killer T cells. These differentiation-inducing factors may be recombinant proteins, natural substances secreted from animal cells, or chemically synthesized products.

The culture medium into which a differentiation-inducing factor is to be incorporated to culture naive T cells is not particularly limited as long as the culture medium is capable of culturing lymphocytes. For example, it is possible to use media such as a Roswell Park Memorial Institute (RPMI) 1640 medium and an Iscove's Modified Dulbecco's Medium (IMDM). These media may be inactivated serum-containing media or serum-free media. In a case where serum is to be incorporated into the media, the concentration of serum is not particularly limited, and can be, for example, 5% to 15% by volume. As the serum, any of fetal bovine serum, bovine serum, horse serum, goat serum, human serum, and the like can also be used. These culture media may appropriately contain additives commonly used as culture media additives such as 2-mercaptoethanol, insulin, transferrin, sodium selenite, ethanolamine, and albumin, in addition to serum.

In the activation step, cells can be cultured in the same manner as in the culture of a normal cell culture strain, except that the cells are cultured in a medium prepared by incorporating a differentiation-inducing factor into a medium capable of culturing lymphocytes. The culture temperature is preferably 30°C to 41°C, and more preferably 36°C to 38°C. The cell culture can be performed in the atmosphere. However, it is preferable to culture the cells in a normal oxygen concentration environment in which the carbon dioxide concentration is controlled to 4% to 10% by volume. The culture time is not particularly limited as long as it is sufficient to activate naive T cells. For example, by culturing the naive T cells for 3 to 28 days, preferably for 6 to 21 days, more preferably for 6 to 14 days, and even more preferably 6 to 10 days, it is possible to activate the naive T cells.

After the activation step, as a memory-formation step, the activated T cells are cultured in a culture medium that does not contain a differentiation-inducing factor in a hypoxic environment. The culture medium that does not contain a differentiation-inducing factor is not particularly limited, and it is possible to use a medium capable of culturing lymphocytes without incorporation of a differentiation-inducing factor. As the medium capable of culturing lymphocytes, it is possible to use an RPMI 1640 medium, an IMDM medium, and the like. Serum or various additives may be added to these basic media. As the additives, the same additives as those listed above can be used. As the culture medium to be used in the memory-formation step, a medium prepared by removing a differentiation-inducing factor from the culture medium used in the activation step may be used, or a basic medium different from the culture medium used in the activation step may be used.

The oxygen concentration during the culture in the memory-formation step is 0.1% to 10% by volume, preferably 0.5% to 5.0% by volume, and more preferably 0.5% to 1.5% by volume. For example, in a low oxygen concentration environment in which the carbon dioxide concentration is controlled to 4% to 10% by volume, the activated T cells are cultured at 30°C to 41°C and preferably at 36°C to 38°C for 5 to 21 days, preferably for 6 to 14 days, and more preferably for 7 to 10 days. By lowering the oxygen concentration to apply stress to the activated T cells, it is possible to form memory conveniently.

By the memory-formation step, the activated T cells differentiate into stem cell memory T cells, central memory T cells, or effector memory T cells. The stem cell memory T cells have self-replication ability and properties of stem cells in addition to long-term viability, and differentiate into central memory T cells or effector memory T cells by antigenic stimulation. The central memory T cells have long-term viability and self-replication ability, and are generally localized in lymph nodes and the peripheral circulatory system. The effector memory T cells have long-term viability, and are generally localized in the peripheral circulatory system and tissue. The stem cell memory T cells, the central memory T cells, and the effector memory T cells can be distinguished based on differences in the expression pattern of cell surface antigens.

While the memory T cells formed ex vivo in the related art are blast cells with irregular shapes and keep performing self-replication, the memory T cells obtained by the memory-formation step in the present invention are small spherical cells just as the natural memory T cells, have a relatively regular shape, and have ceased expansion. Based on the morphological characteristics and the characteristics of expansion cessation/quiescence of cells described above, it can be said that the manufacturing method of memory T cells according to the present invention is a method which makes it possible to conveniently obtain memory T cells having traits close to those of natural memory T cells.

By the memory-formation step, a cell population is obtained in which a proportion of memory cells in all living cells is 20% or more, preferably 50% or more, more preferably 70% or more, and even more preferably 80% or more. That is, the culture obtained after culturing in the memory-formation step is a memory T cell-containing composition in which the total number of living cells including stem cell memory T cells, central memory T cells, and effector memory T cells is equal to or more than 20%, preferably equal to or more than 50%, more preferably equal to or more than 70%, and even more preferably equal to or more than 80% of the total number of living cells contained in the composition.

By using naive cells pretreated with stress instead of applying hypoxic stress in the memory-formation step or by performing a stress treatment on the activated T cells before the memory-formation step, it is possible to induce cellular quiescence and to form the naive T cells into memory. Examples of the stress treatment include temperature stress and aging.

Specifically, by applying temperature stress to the naive T cells before the memory-formation step, it is possible to form memory. Examples of the temperature stress include low-temperature stress at 0°C to 10°C and preferably at 1 °C to 5°C, and freezing stress.

For example, the naive T cells to be activated with a differentiation-inducing factor in the activation step are subjected to a cold storage treatment in which the cells are kept at 0°C to 10°C for 1 hour or longer, preferably for 1 to 7 days, and more preferably for 2 to 4 days to apply low-temperature stress. By activating the naive T cells after exposure to the low-temperature stress with a differentiation-inducing factor and then culturing the activated T cells in the absence of a differentiation-inducing factor, it is possible to manufacture memory T cells. The activated T cells may be cultured in a culture medium that does not contain a differentiation-inducing factor, in a normal oxygen environment or in a hypoxic environment.

In addition, for example, by performing a freeze-thaw treatment on the naive T cells not yet being subjected to the activation step of activating the cells with a differentiation-inducing factor, then activating the T cells with a differentiation-inducing factor, and then culturing the activated T cells in the absence of a differentiation-inducing factor, it is also possible to manufacture memory T cells. The freeze-thaw can be performed in the same manner as a general freeze-thaw for cells. For example, by suspending the cells in a cryopreservation solution and leaving the obtained suspension to stand in a freezer at -70°C to -150°C, it is possible to freeze the naive T cells. The cryopreservation solution may be the medium used for cell culture, a solution obtained by adding serum to a buffer such as phosphate-buffered saline (PBS), or a solution obtained by adding a cell protectant such as DMSO or sericin. Some cell protectants must be added, but serum may not be added. It is possible to thaw the frozen cells by using a water bath or the like.

The temperature stress may be applied before the activated T cells are formed into memory. For example, by freezing and thawing the activated T cells after the activation step and then culturing the T cells in the absence of a differentiation-inducing factor as a memory-formation step, it is possible to manufacture memory T cells. The T cells after freeze-thaw may be cultured in a culture medium that does not contain a differentiation-inducing factor, in a normal oxygen environment or a hypoxic environment.

In a case where naive T cells exposed to aging stress are used, memory T cells can be manufactured without application of hypoxic stress or temperature stress. For example, in the case of naive T cells collected from a human aged 50 or over, by activating the naive T cells with a differentiation-inducing factor and then culturing the cells in a culture medium that does not contain a differentiation-inducing factor in a normal oxygen environment, it is possible to form memory.

Just as other memory T cells, the memory T cells obtained by memory formation or a composition containing the memory T cells can be used as an active ingredient of a pharmaceutical composition. Furthermore, the memory T cells can be used as a raw material of cells to be administered to patients as a pharmaceutical product in cell therapy and the like. For example, by genetically modifying the memory T cells obtained by memory formation, it is possible to manufacture CAR-T cells used in CAR-T therapy.

In addition, in DC vaccine therapy, returning the memory T cells obtained by memory formation back into the patient's body together with activated cancer cell surface antigen-presenting cells could bring about a higher therapeutic effect. For example, a part of the fraction containing activated lymphocytes collected simultaneously with the manufacturing of a DC vaccine is subjected to a freezing treatment, then thawed, and then cultured in a differentiation-inducing factor-free medium in a hypoxic environment. A cancer antigen-specific lymphocyte population having formed memory, which is obtained by mixing surviving cells after memory formation with activated cancer cell surface antigen-presenting cells, is returned back to the patient's body.

For T cells other than naive T cells, memory can also be formed by culturing the T cells in a culture medium that does not contain a differentiation-inducing factor in a hypoxic environment. For example, in a case where CAR-T cells prepared for being used in CAR-T therapy are cultured in a culture medium that does not contain a differentiation-inducing factor in a hypoxic environment, CAR-T cells having formed memory, that is, CAR-T cells having undifferentiated and quiescent long-term viability are obtained.

In addition, the manufacturing method of memory T cells according to the present invention can also be used to solve T cell dysfunction, which is an issue of cancer immunotherapy. T cell dysfunction is a phenomenon where T cells that attack cancer cells are continuously exposed to cancer antigens for an excessively long period of time and leads to the reduction of the ability to attack cancer cells. A plurality of transcription factors, such as TOX, TOX2, and NR4A, genomic modification, metabolic change, and the like are known to be involved in such dysfunction. In a case where T cells dysfuncted by receiving excessive activation stimulation due to long-term exposure to cancer antigens are cultured in a culture medium that does not contain a differentiation-inducing factor in a hypoxic environment, the factors causing T cell dysfunction are likely to be downregulated, and the intracellular conditions of the T cell are likely to be reset. In a case where the T cells having formed memory obtained in this way are returned back into the patient's body, a therapeutic effect that lasts longer in the cancer immunotherapy is obtained.

### <Manufacturing method of undifferentiated and quiescent cells viable for a long period of time independently of differentiation-inducing factor from naive B cells>

For naive B cells, by activating the cells and then culturing the cells under hypoxic stress, it is possible to obtain undifferentiated and quiescent cells having a trait of being viable for a long period of time independently of a differentiation-inducing factor. Specifically, a manufacturing method of cells having such a trait includes an activation step of activating naive B cells with a differentiation-inducing factor and a long-term viability acquisition step of culturing the activated B cells in the absence of a differentiation-inducing factor after the activation step to manufacture cells viable independently of a differentiation-inducing factor, in which the activated B cells are cultured in a hypoxic environment in the long-term viability acquisition step.

The naive B cells used in the present invention may be either naive B cells collected from an animal or cells obtained by expanding naive B cells collected from an animal by primary culture of subculture. In addition, in the present invention, it is also possible to use naive B cells differentiated from embryonic stem (ES) cells and induced pluripotent stem (iPS) cells.

The collection of naive B cells from a living body can be performed by purification from PBMC using FACS, in the same manner as for the naive T cells. For example, the naive B cells have CD20 and CD27 among cell surface antigens, but do not have CD38. Therefore, by labeling PBMC with fluorescently labeled antibodies against these cell surface antigens and then using FACS, it is possible to purify only a naive B cell population that expresses CD20 and CD27 and does not express CD38. The FACS device and the fluorescently labeled antibodies against various surface antigens to be used can be appropriately selected from those which are generally used, and commercially available products can also be used. In addition, instead of FACS, immunomagnetic beads composed of magnetic beads bound to antibodies against the aforementioned cell surface antigens can also be used to purify naive T cells from PBMC by using a magnet.

The differentiation-inducing factor to be used in the activation step of activating naive B cells can be appropriately selected from cytokines necessary for inducing differentiation of naive B cells into effector cells. For example, it is possible to activate the naive B cells to differentiate into plasma cells by using IL-2, IL-4, IL-6, IL-10, IL-15, and IL-21.

In the activation step of activating the naive B cells with a differentiation-inducing factor, as the culture medium, a medium prepared by incorporating a differentiation-inducing factor into a medium capable of culturing lymphocytes is used. As the medium capable of culturing lymphocytes, it is possible to use an RPMI 1640 medium, an IMDM medium, and the like. Serum or various additives may be added to these basic media. As the additives, the same additives as those listed above can be used.

In the activation step, cells can be cultured in the same manner as in the culture of a normal cell culture strain, except that the cells are cultured in a medium prepared by incorporating a differentiation-inducing factor into a medium capable of culturing lymphocytes. The culture temperature is preferably 30°C to 41°C, and more preferably 36°C to 38°C. The cell culture can be performed in the atmosphere. However, it is preferable to culture the cells in a normal oxygen concentration environment in which the carbon dioxide concentration is controlled to 4% to 10% by volume. The culture time is not particularly limited as long as it is sufficient to activate naive B cells. For example, by culturing the naive B cells for 2 to 28 days, preferably for 3 to 21 days, more preferably for 3 to 14 days, and even more preferably for 3 to 10 days, it is possible to activate the naive B cells.

After the activation step, as a long-term viability acquisition step, the activated B cells are cultured in a culture medium that does not contain a differentiation-inducing factor in a hypoxic environment. The culture medium that does not contain a differentiation-inducing factor is not particularly limited, and it is possible to use a medium capable of culturing lymphocytes without incorporation of a differentiation-inducing factor. As the medium capable of culturing lymphocytes, it is possible to use an RPMI 1640 medium, an IMDM medium, and the like. Serum or various additives may be added to these basic media. As the additives, the same additives as those listed above can be used. As the culture medium to be used in the long-term viability acquisition step, a medium prepared by removing a differentiation-inducing factor from the culture medium used in the activation step may be used, or a basic medium different from the culture medium used in the activation step may be used.

The oxygen concentration during the culture in the long-term viability acquisition step is 0.1 % to 10% by volume, preferably 0.5% to 5% by volume, and more preferably 0.5% to 1.5% by volume. For example, in a low oxygen concentration environment in which the carbon dioxide concentration is controlled to 4% to 10% by volume, the activated B cells are cultured at 30°C to 41 °C and preferably at 36°C to 38°C for 5 to 21 days, preferably for 6 to 14 days, and more preferably for 6 to 10 days. By lowering the oxygen concentration to apply stress to the activated B cells, it is possible to conveniently obtain long-term viability independent of a differentiation-inducing factor.

By the long-term viability acquisition step, the activated B cells acquire long-term viability independent of a differentiation-inducing factor while remaining as plasma cells, or differentiate into germinal center B cells, transitional B cells, and the like. Both the germinal center B cells and transitional B cells have long-term viability independent of a differentiation-inducing factor.

Just as other B cells, the B cells that have acquired long-term viability independent of differentiation-inducing factors or a composition containing such B cells can be used as an active ingredient of a pharmaceutical composition. Furthermore, the B cells having acquired long-term viability independent of a differentiation-inducing factor can be used as a raw material of cells to be administered to patients as a pharmaceutical product in cell therapy and the like.

In the body of an animal, natural long living plasma cells are localized in the bone marrow, which is a hypoxic environment, and keep secreting antibodies into the blood throughout life regardless of the presence or absence of antigenic stimulation or cytokines. Therefore, by transplanting the long living plasma cells that produce antibodies against specific antigens, it is possible to provide humoral immunity to an individual animal without vaccination. Just as natural long living plasma cells, the plasma cells (long-lived plasma cells) prepared by the present invention that have acquired long-term viability independent of a differentiation-inducing factor can provide humoral immunity to an individual animal by imparting antibody producibility to the animal. For example, because the transplantation of long-lived plasma cells prepared from naive B cells stimulated by a specific antigen can induce immunity against the antigen, the present invention is expected to be effective for preventing or treating infections for which no effective vaccines have been developed, such as HIV virus, enterohemorrhagic E. coli (such as 0157), and SARS-CoV-2, which is the cause of COVID-19.

### [Examples]

Next, the present invention will be more specifically described with reference to examples and the like, but the present invention is not limited to these examples.

### [Culture medium and the like]

The compositions of the media used in the following experiment were as follows.

PBS/2% FBS: A solution prepared by mixing 49 mL of phosphate-buffered saline (PBS) with 1 mL of fetal bovine serum (FBS).

PBS/2% FBS/1 mM EDTA: A solution prepared by mixing 49 mL of PBS with 1 mL of FBS and 0.1 mL of 0.5 M EDTA.

RPMI 1640/10% FBS/2-ME: A medium prepared by mixing 45 mL of RPMI 1640 (#12633-012, manufactured by Invitrogen) with 5 mL of FBS, 0.5 mL of 100× PSG ("100× penicillin-streptomycin-glutamine", #10378-016, manufactured by Invitrogen), and 50 µL of 50 mM 2-mercaptoethanol (2-ME).

IMDM/10% FBS/2-ME: A medium prepared by mixing 45 mL of IMDM ("IMDM (×1) + GlutaMAX-1 medium", #31980-030, manufactured by Invitrogen) with 5 mL of FBS, 0.5 mL of 100× PSG, and 50 µL of 50 mM 2-ME.

Th1 medium: A medium prepared by mixing 10 mL of RPMI 1640/10% FBS/2-ME with 50 µL of "200× Human Th1 reagent 1 (IL-12)" and 50 µL of "200x Human Th1 reagent 2 (IFN-γ)". As 200× Human Th1 reagent 1 (IL-12) and 200x Human Th1 reagent 2 (IFN-γ), the adjuncts of a Th1 differentiation induction kit ("CellXVivo human Th1 cell differentiation kit". #CDK001, manufactured by R & D Systems) were used.

Th2 medium: A medium prepared by mixing 10 mL of RPMI 1640/10% FBS/2-ME with 10 µL of "1000× Human Th2 reagent 1 (IL-2)", 10 µL of "1000× Human Th2 reagent 2 (IL-4)", 10 mL of "1000× Human Th2 reagent 3 (IL-7)", and 10 µL of "1000x Human Th2 reagent 4 (TSLP)". As 1000× Human Th2 reagent 1 (IL-2), 1000× Human Th2 reagent 2 (IL-4), 1000× Human Th2 reagent 3 (IL-7), and 1000x Human Th2 reagent 4 (TSLP), the adjuncts of a Th2 differentiation induction kit ("CellXVivo human Th2 cell differentiation kit", #CDK002, manufactured by R & D Systems) were used.

CD8-TA medium: A medium prepared by mixing 10 mL of RPMI 1640/10% FBS/2-ME with 10 µL of T cell activator magnetic beads ("Dynabeads human T-activator CD3/CD28", #11131D, manufactured by Gibco) and 10 µL of an IL-2 (human recombinant IL-2, #202-1L, manufactured by R & D Systems) solution (10 µg/mL).

Step 1 medium: A medium prepared by mixing 10 mL of RPMI 1640/10% FBS/2-ME with 20 µL of "500× B cell Expander 1 (CD40L)", 20 µL of "500× B cell Expander 2 (IL-4)", 20 µL of "500× B cell Expander 3 (anti-CD40L)", 10 µL of an IL-2 (human recombinant IL-2, #202-IL, manufactured by R & D Systems) solution (10 µg/mL), 50 µL of an 1L-10 (human recombinant IL-10, #217-1L-005, manufactured by R & D Systems) solution (10 µg/mL), and 50 µL of an IL-21 (human recombinant IL-21, #200-21-10UGR, manufactured by PeproTech, Inc.) solution (10 µg/mL). As 500× B cell Expander 1 (CD40L), 500× B cell Expander 2 (1L-4), and 500× B cell Expander 3 (anti-CD40L), the adjuncts of a B cell expansion kit ("CellXVivo human B cell expansion kit", #CDK005, manufactured by R & D Systems) were used.

Step 2 medium: A medium prepared by mixing 10 mL of IMDM/10% FBS/2-ME with 10 µL of an IL-2 solution (10 µg/mL), 5 µL of an IL-6 (human recombinant 1L-6, #200-06-20UG, (manufactured by PeproTech, Inc.) solution (20 µg/mL), and 50 µL of an IL-21 solution (10 µg/mL).

B27 medium: A solution prepared by mixing 48.5 mL of RPMI 1640 with 1 mL of "50× B27 supplement" (serum free, #17504044, manufactured by Gibco), 0.5 mL of 100× PSG, and 50 µL of 50 mM 2-ME.

N2 medium: A solution prepared by mixing 49 mL of RPMI 1640 with 0.5 mL of "100× N2 supplement" (#17502001, manufactured by Gibco), 0.5 mL of 100× PSG, and 50 µL of 50 mM 2-ME.

BSA medium: A solution prepared by mixing 49 mL of RPMI 1640 with 0.5 mL of 100× BSA (200 mg/mL, #A4161, manufactured by Sigma-Aldrich Co., LLC.), 0.5 mL of 100× PSG, and 50 µL of 50 mM 2-ME.

LPS/IL-4 medium: A medium prepared by mixing 10 mL of RPMI 1640/10% FBS/2-ME with 50 µL of a lipopolysaccharide (LPS) (E. coli OIII: B4, L4391-1MG, manufactured by Sigma-Aldrich Co. LLC.) solution (1 mg/mL) and 10 µL of an IL-4 (human recombinant IL-4, AF-200-04, manufactured by PeproTech, Inc.) solution (10 µg/mL).

ODN2006 medium: A medium prepared by mixing 10 mL of RPMI 1640/10% FBS/2-ME with 10 µL of an IL-2 solution (10 µg/mL), 5 µL of an IL-6 (human recombinant IL-6, #200-06-20UG, manufactured by PeproTech, Inc.) solution (20 µg/mL), and 20 µL of an oligonucleotide 2006 (ODN2006) (tlrl-2006, manufactured by InvivoGen) solution (2.5 mg/mL).

### [Antibodies and the like used for FACS]

The antibodies and the like used in FACS were as follows.
Anti-CD45RA antibody: FITC-labeled mouse anti-human CD45RA monoclonal antibody (H1100, #11-0458-42, manufactured by Invitrogen)
Anti-CD62L antibody: PE-labeled mouse anti-human CD62L monoclonal antibody (DREG-56, #12-0629-42, manufactured by Invitrogen)
Anti-CD127 antibody: PE-Cyanine 7-labeled mouse anti-human CD127 monoclonal antibody (eBioRDR5, #25-1278-42, manufactured by Invitrogen)
Anti-CD20 antibody: FITC-labeled mouse anti-human CD20 monoclonal antibody (2H7, #11-0209-42, manufactured by Invitrogen)
Anti-CD27 antibody: PE-labeled mouse anti-human CD27 monoclonal antibody (0323, #12-0279-42, manufactured by Invitrogen)
Anti-CD38 antibody: PE-Cyanine 7-labeled mouse anti-human CD38 monoclonal antibody (HIT2, #25-0389-42, manufactured by Invitrogen)
7-AAD: 7-Amino-Actinomycin D (#559925, manufactured by BD Biosciences)

### [Example 1]

Differentiation of naive CD4⁺ T cells recovered from peripheral blood collected from a 21-year-old human was induced, and then the cells were cultured in a differentiation-inducing factor deficient medium in a hypoxic environment, thereby manufacturing memory lymphocytes.

### (1) Collection of human peripheral blood mononuclear cells (PBMC)

Blood collected for two heparin-containing blood collection tubes (about 20 mL) was put in a 50 mL plastic tube, and 20 mL of PBS/2% FBS was added thereto for 2X dilution, followed by mixing by performing pipetting about 10 times with a pipette having a volume of 10 mL with care to prevent bubbling.

The blood diluted as above was layered by a volume of 20 mL in each of two PBMC recovery tubes ("SepMate (registered trademark)-50", #86450, manufactured by STEMCELL Technologies.) into which 15 mL of Ficoll was put in advance, such that the Ficoll layer was not disturbed. Next, the PBMC recovery tube was centrifuged (2,600 rpm, 10 minutes, room temperature). Thereafter, the contents of the PBMC recovery tube were moved by decantation to a plastic tube having a volume of 50 mL into which 25 mL of PBS/2% FBS was put in advance, followed by centrifugation (1,300 rpm, 8 minutes, room temperature). The supernatant of the plastic tube was removed, the precipitated cell pellets were dispersed by tapping, 50 mL of PBS/2% FBS was then added thereto by decantation, the tube was inverted 3 times for mixing, and centrifugation was then performed again (1,300 rpm, 8 minutes, room temperature) to remove the supernatant. PBS/2% FBS/1 mM EDTA (10 mL) was added to the cell pellet precipitated in the plastic tube, followed by performing pipetting about 10 times to suspend the cells.

In order to count the number of cells, 10 µL of the obtained cell suspension was separated to another tube. The same amount of Turk's solution (nuclear staining solution: #109277, manufactured by Sigma-Aldrich Co., LLC.) was added to 10 µL of the cell suspension, and the number of nucleated cells was calculated.

PBS/2% FBS/1 mM EDTA (40 mL) was added to the entirety of the remaining cell suspension such that the total amount was adjusted to 50 mL, and the tube was inverted 3 times for mixing, followed by centrifugation (1,300 rpm, 8 minutes, room temperature) to remove the supernatant. A buffer for cell separation ("RoboSep (registered trademark) Buffer", #20104, manufactured by STEMCELL Technologies.) was added to the cell pellets precipitated in the plastic tube, thereby preparing a cell suspension at 5 × 10⁷ cells/mL.

### (2) Recovery of naive CD4⁺ T cells from PBMC

Naive CD4⁺ T cells were recovered from the cell suspension prepared in (1) described above. The naive CD4⁺ T cells were recovered using a commercially available naive CD4⁺ T cell isolation kit ("EasySep human naive CD4⁺ T cell isolation kit II", #17555, manufactured by STEMCELL Technologies.) according to the manual attached to the kit.

From the purified naive CD4⁺ T cell suspension, a portion having a volume of 10 µL was separated to another tube for counting the number of cells. The same amount of a trypan blue solution (#29853-34, manufactured by Nacalai Tesque Inc.) was added to 10 µL of the cell suspension, and the number of living cells was calculated.

### (3) Induction of differentiation of naive CD4⁺ T cells into CD4⁺ Th1 cells

The differentiation of naive CD4⁺ T cells into CD4⁺ Th1 cells was induced using a commercially available Th1 differentiation induction kit ("CellXVivo human Th1 cell differentiation kit", #CDK001, manufactured by R & D Systems) according to the manual attached to the kit.

First, the naive CD4⁺ T cell suspension prepared and purified in (2) described above was moved to a plastic tube having a volume of 15 mL and then centrifuged (1,000 rpm, 5 minutes, room temperature) to remove the supernatant. A Th1 medium was added to the cell pellets precipitated in the plastic tube such that the cell density was 2 × 10⁵ cells/mL, thereby suspending the cells.

The obtained cell suspension was dispensed at 1 mL/well into a 24-well plate coated with a mouse anti-human CD3 antibody (adjunct of the kit). The plate was cultured for 7 days or longer in an environment of 37°C, 5% CO₂, and 20% O₂. In a case where the pH of the medium went through pH reduction or turned yellow due to the lactic acid generated by the increase in the number cells during the culture period, half the amount of the culture solution was moved to other mouse anti-human CD3 antibody-coated well and diluted 2X with a fresh Th1 medium, and culture was continued.

### (4) Induction of differentiation of naive CD4⁺ T cells into CD4⁺ Th2 cells

The differentiation of naive CD4⁺ T cells into CD4⁺ Th2 cells was induced using a commercially available Th2 differentiation induction kit ("CellXVivo human Th2 cell differentiation kit", #CDK002, manufactured by R & D Systems) according to the manual attached to the kit.

First, the naive CD4⁺ T cell suspension prepared and purified in (2) described above was moved to a plastic tube having a volume of 15 mL and then centrifuged (1,000 rpm, 5 minutes, room temperature) to remove the supernatant. A Th2 medium was added to the cell pellets precipitated in the plastic tube such that the cell density was 2 × 10⁵ cells/mL, thereby suspending the cells.

The obtained cell suspension was dispensed at 1 mL/well into a 24-well plate coated with a mouse anti-human CD3 antibody (adjunct of the kit). The plate was cultured for 7 days or longer in an environment of 37°C, 5% CO₂, and 20% O₂. In a case where the medium went through pH reduction or turned yellow due to the lactic acid generated by the increase in the number of cells during the culture period, half the amount of the culture solution was moved to other mouse anti-human CD3 antibody-coated wells and diluted 2X with a fresh Th2 medium, and culture was continued.

### (5) Culture in differentiation-inducing factor deficient medium

After the culture in (3) described above finished, for each culture solution, the cells in each well were moved to a plastic tube having a volume of 15 mL. From the culture solution moved to the plastic tube, a portion having a volume of 10 µL was separated to another tube for counting the number of cells. The same amount of a trypan blue solution was added to 10 µL of the cell suspension, and the number of living cells was calculated.

A plastic tube containing the remaining cell culture solution was centrifuged (1,000 rpm, 5 minutes, room temperature) to remove the supernatant, and then RPMI 1640/10% FBS/2-ME was added to the precipitated cell pellets such that the cell density was 4 × 10⁵ cells/mL and the cells were suspended, thereby preparing a cell suspension.

The prepared cell suspension was dispensed into T25 flasks in 5 mL portions. These T25 flasks were cultured (hypoxic culture) for 7 days or longer in an environment of 37°C, 5% CO₂, and 1% O₂. Control cells were cultured (normal culture) for 7 days or longer in an environment of 37°C, 5% CO₂, and 20% O₂.

The cultured cells were subjected to FACS analysis.

The cells obtained after the completion of the culture in (4) described above were also cultured in a hypoxic environment, and then the cultured cells were subjected to FACS analysis.

### (6) FACS

For each culture solution, the cells obtained after the culture in (5) described above were moved to a plastic tube having a volume of 15 mL. The plastic tube was centrifuged (1,000 rpm, 5 minutes, room temperature) to remove the supernatant, and then 5 mL of PBS/2% FBS was added to the precipitated cell pellets to suspend the cells. Thereafter, the plastic tube was centrifuged (1,000 rpm, 5 minutes, room temperature) to remove the supernatant. PBS/2% FBS (0.1 mL) was added to the precipitated cell pellets to suspend the cells.

An antibody cocktail for T cells (22 µL, 5 µL of anti-CD45RA antibodies, 5 µL of anti-CD62L antibodies, 5 µL of anti-CD127 antibodies, and 7 µL of 7-AAD) was added to the prepared cell suspension, followed by pipetting for mixing, and then the mixture was left to stand on ice in a dark place for 20 minutes. Next, 5 mL of PBS/2% FBS was added to the cell suspension to suspend the cells, and the plastic tube was centrifuged (1,000 rpm, 5 minutes, room temperature) to remove the supernatant. PBS/2% FBS (0.5 mL) was added to the precipitated cell pellets to suspend the cells, and the obtained cell suspension was moved to a FACS tube having a volume of 5 mL.

The FACS tube was set in a FACS device ("FACSCanto 11", manufactured by Becton, Dickinson and Company.) and measured, and the obtained data was analyzed using analysis software ("FACSDiva ver. 6.1", manufactured by Becton, Dickinson and Company.).

The naive T cells (naive CD4⁺ T cells and naive CD8 ⁺ T cells), effector T cells (CD4⁺ Th1 cells, CD4⁺ Th2 cells, and CD8⁺ killer T cells), stem cell memory T cells (Stem Cell Memory T Cells), central memory T cells, and effector memory T cells each had the cell surface antigens shown in Table 1.

**[Table 1]**

| Cell | Surface antigen |
|---|---|
| Naive T cells | CD45RA+ / CD62L+ / CD127+ |
| Stem cell-like memory T cells | CD45RA+ / CD62L+ / CD127+ |
| Central memory T cells | CD45RA- / CD62L+ / CD127+ |
| Effector memory T cells | CD45RA- / CD62L- / CD127+ |
| Effector T cells | CD45RA- / CD62L- / CD127- |

FIG. 1(A) is a dot plot showing the results of FACS on naive CD4⁺ T cells (cells before stimulation) before differentiation induction, FIG. 1(B) is a dot plot showing the results of FACS on cells cultured in a Th1 medium for 15 days (cells under stimulation for 15 days), and FIGS. 1(C) and 1(D) are dot plots showing the results of FACS on cells cultured for 13 days in a Th1 medium and then cultured in RPM11640/10% FBS/2-ME (differentiation-inducing factor deficient medium) for 9 days in a hypoxic environment (cells under stimulation for 13 days + cells under no stimulation for 9 days). In a case where the naive CD4⁺ T cells were cultured in a Th1 medium for 13 days and then cultured in a differentiation-inducing factor deficient medium for 9 days in a normal environment, death of all cells was confirmed by FACS analysis.

In a case where the naive CD4⁺ T cells were cultured in a Th1 medium for 15 days, it was confirmed that most of the cells were CD45RA⁻ cells, and that a mixed population of mature effector cells (CD4⁺ Th1 cells) and immature effector cells was formed (FIG. 1(B)). On the other hand, in a case where the naive CD4⁺ T cells were cultured in the Th1 medium for 13 days and then cultured in a differentiation-inducing factor deficient medium for 9 days in a hypoxic environment, unlike the normal culture, 52.0% of the cells survived. Furthermore, it was confirmed that this cell population was a cell population consisting of stem cell memory T cells, central memory T cells, and effector memory T cells (FIGS. 1(C) and 1(D)).

FIG. 2(A) is a dot plot showing the results of FACS on naive CD4⁺ T cells (cells before stimulation) before differentiation induction, FIG. 2(B) is a dot plot showing the results of FACS on cells cultured in a Th2 medium for 17 days (cells under stimulation for 17 days), and FIGS. 2(C) and 2(D) are dot plots showing the results of FACS on cells cultured for 10 days in a Th2 medium and then cultured in RPMI 1640/10% FBS/2-ME (differentiation-inducing factor deficient medium) for 7 days in a hypoxic environment (cells under stimulation for 10 days + cells under no stimulation for 7 days). In a case where the naive CD4⁺ T cells were cultured in a Th2 medium for 10 days and then cultured in a differentiation-inducing factor deficient medium for 7 days in a normal environment, death of all cells was confirmed by FACS analysis.

In a case where the naive CD4⁺ T cells were cultured in a Th2 medium for 17 days, it was also confirmed that most of the cells were CD45RA⁻ cells, and that a mixed population of mature effector cells (CD4⁺ Th2 cells) and immature effector cells was formed (FIG. 2(B)). On the other hand, in a case where the naive CD4⁺ T cells were cultured in the Th2 medium for 10 days and then cultured in a differentiation-inducing factor deficient medium for 7 days in a hypoxic environment, unlike the normal culture, 32.6% of the cells survived. Furthermore, it was confirmed that this cell population was a cell population consisting of stem cell memory T cells, central memory T cells, and effector memory T cells (FIGS. 2(C) and 2(D)). However, unlike the cell suspension cultured in the Th1 medium, in this cell suspension, the number of effector memory T cells was extremely small, and many stem cell memory T cells were generated.

From the results shown in FIGS. 1 and 2, it was confirmed that activating naive CD4⁺ T cells by differentiation induction and then culturing the cells in a hypoxic environment makes it possible to convert the cells into memory cells having acquired long-term viability and to generate not only central memory T cells and effector memory T cells but also stem cell memory T cells a having self-replication ability and properties of stem cells.

### (7) Hypoxic culture in serum-free medium

The culture in (5) described above was performed using three serum-free media of a B27 medium, an N2 medium, and a BSA medium, instead of RPMI 1640/10% FBS/2-ME. FACS analysis was performed on the cultured cells in the same manner as in (6) described above.

As a result, in a case where the cells were cultured in a Th1 medium or a Th2 medium for 10 days and then cultured in a B27 medium, an N2 medium, or a BSA medium for 7 days, a cell population consisting of stem cell memory T cells, central memory T cells, and effector memory T cells was obtained. That is, in all the cases using serum-free media, it was possible to manufacture memory cells having acquired long-term viability, just as the case using RPMI 1640/10% FBS/2-ME.

### [Example 2]

Differentiation of naive CD8⁺ T cells recovered from peripheral blood collected from a 20-year-old human was induced, and then the cells were cultured in a differentiation-inducing factor deficient medium in a hypoxic environment, thereby manufacturing memory lymphocytes.

### (1) Recovery of naive CD8⁺ T cells from PBMC

Naive CD8⁺ T cells were recovered from a cell suspension prepared in the same manner as in (1) of Example 1. The naive CD8⁺ T cells were recovered using a commercially available naive CD8⁺ T cell isolation kit ("EasySep human naive CD8⁺ T cell isolation kit II", #17928, manufactured by STEMCELL Technologies.) according to the manual attached to the kit.

From the purified naive CD8⁺ T cell suspension, a portion having a volume of 10 µL was separated to another tube for counting the number of cells. The same amount of a trypan blue solution was added to 10 µL of the cell suspension, and the number of living cells was calculated.

### (2) Induction of differentiation of naive CD8⁺ T cells into CD8⁺ killer T cells

First, the naive CD8⁺ T cell suspension prepared and purified in (1) described above was moved to a plastic tube having a volume of 15 mL and then centrifuged (1,000 rpm, 5 minutes, room temperature) to remove the supernatant. A CD8-TA medium was added to the cell pellets precipitated in the plastic tube such that the cell density was 2 × 10⁵ cells/mL, thereby suspending the cells.

The obtained cell suspension was dispensed at 1 mL/well to an uncoated 24-well plate. The plate was cultured for 7 days or longer in an environment of 37°C, 5% CO₂, and 20% O₂. In a case where the medium went through pH reduction or turned yellow due to the lactic acid generated by the increase in the number of cells during the culture period, half the amount of the culture solution was moved to other mouse anti-human CD3 antibody-coated wells and diluted 2X with a fresh Th2 medium, and culture was continued.

### (3) Removal of Magnetic Beads

After the culture in (2) described above finished, for each culture solution, the cells in each well were moved to a plastic tube having a volume of 15 mL. The plastic tube was centrifuged (1,000 rpm, 5 minutes, room temperature) to remove the supernatant, and then 0.8 mL of a cell separation buffer "RoboSep Buffer" was added to the precipitated cell pellets to suspend the cells. Thereafter, the suspension was vigorously pipetted for T cell activation and removal of magnetic beads, and 1.7 mL of the cell separation buffer was further added thereto, thereby preparing 2.5 mL of a cell suspension.

The cell suspension was moved to a FACS tube having a volume of 5 mL (#720028, manufactured by Corning Incorporated), and the FACS tube was set on a magnetic stand and left to stand at room temperature for 3 minutes. Subsequently, in a state where the FACS tube was set on the magnetic stand, the cell suspension in the FACS tube was moved by decantation to a new FACS tube having a volume of 5 mL. The new FACS tube was set on a magnetic stand again and left to stand at room temperature for 3 minutes, and then the cell suspension in the FACS tube was moved by decantation to a new FACS tube having a volume of 5 mL.

### (4) Culture in differentiation-inducing factor deficient medium

From the cell suspension recovered into the FACS tube in (3) described above, a portion having a volume of 10 µL was separated to another tube for counting the number of cells. The same amount of a trypan blue solution was added to 10 µL of the cell suspension, and the number of living cells was calculated.

The entirety of the cell culture solution remaining in the FACS tube was moved to a plastic tube having a volume of 15 mL and then centrifuged (1,000 rpm, 5 minutes, room temperature) to remove the supernatant. RPM11640/10% FBS/2-ME was added to the cell pellets precipitated in the plastic tube such that the cell density was 4 × 10⁵ cells/mL and the cells were suspended, thereby preparing a cell suspension.

The prepared cell suspension was dispensed into T25 flasks in 5 mL portions. These T25 flasks were cultured (hypoxic culture) for 7 days in an environment of 37°C, 5% CO₂, and 1% O₂. Control cells were cultured (normal culture) for 7 days in an environment of 37°C, 5% CO₂, and 20% O₂.

The cultured cells were subjected to FACS analysis.

### (5) FACS

FACS analysis was performed on the cells obtained after the culture in (4) described above, in the same manner as in (6) of Example 1.

FIG. 3 is a view showing the results of FACS analysis on cells obtained by culturing naive CD8⁺ T cells in a CD8-TA medium and then in a differentiation-inducing factor deficient medium in a hypoxic environment. FIGS. 3(A) and 3(D) are dot plots showing the results of FACS on naive CD8⁺ T cells (cells before stimulation) before differentiation induction, FIGS. 3(B) and 3(E) are dot plots showing the results of FACS on cells cultured in a CD8-TA medium for 8 days (cells under stimulation for 8 days), and FIGS. 3(C) and 3(F) are dot plots showing the results of FACS on cells cultured for 7 days in a CD8-TA medium and then cultured in RPMI 1640/10% FBS/2-ME (differentiation-inducing factor deficient medium) for 8 days in a hypoxic environment (cells under stimulation for 7 days + cells under no stimulation for 8 days). In FIG. 3, (A) to (C) on the upper side are graphs in which side scatter signals (SSC) are plotted on the ordinate and forward scatter signals (FSC) are plotted on the abscissa, and (D) to (F) on the lower side are graphs in which the intensity of fluorescence emitted from the anti-CD62L antibody is plotted on the ordinate and the intensity of fluorescence emitted from the anti-CD45RA antibody is plotted on the abscissa. In addition, in the plots on the upper sides, the cells within the gated region are living cells.

In a case where the naive CD8⁺ T cells were cultured in a CD8-TA medium for 7 days and then cultured in a differentiation-inducing factor deficient medium for 8 days in a normal environment, death of all cells was confirmed by FACS analysis. On the other hand, in a case where the naive CD8⁺ T cells were cultured in a CD8-TA medium for 7 days and then cultured in a differentiation-inducing factor deficient medium for 8 days in a hypoxic environment, unlike the normal culture, 20.0% of the cells survived. In addition, according to the expression pattern of surface antigens, the cell population obtained after culture in a differentiation-inducing factor deficient medium in a hypoxic environment was mainly composed of stem cell memory T cells, central memory T cells were the second most abundant cells in the population, and the number of effector memory T cells in the population was extremely small (FIG. 3(F)).

In addition, as shown in the plots on the upper side in FIG. 3, the naive CD8⁺ T cells had a relatively regular shape and were small (FIG. 3(A)), CD8⁺ T cells activated by differentiation induction had a wide variation in shape, and most of the cells were larger than the naive CD8⁺ T cells (FIG. 3(B)). On the other hand, a cell population cultured in a differentiation-inducing factor deficient medium in a hypoxic environment after activation had a relatively regular shape and was smaller than the activated cells (FIG. 3(C)). That is, the cells obtained after the culture in a differentiation-inducing factor deficient medium in a hypoxic environment were more similar to effector T cells compared to natural memory T cells, not only in the expression pattern of cell surface antigens but also in shape and size.

From these results, it was confirmed that activating naive CD8⁺ T cells by differentiation induction and then culturing the cells in a differentiation-inducing factor deficient medium in a hypoxic environment makes it possible to obtain memory cells having acquired long-term viability and to extremely efficiently manufacture stem cell memory T cells a having self-replication ability and properties of stem cells.

### [Example 3]

Naive CD4+ T cells recovered from peripheral blood collected from a 21-year-old human were induced to differentiate into CD4+ Th2 cells, and then the cells were frozen and thawed, thereby manufacturing memory lymphocytes. The recovery of the naive CD4⁺ T cells from human peripheral blood and the induction of differentiation of the naive CD4⁺ T cells into CD4⁺ Th2 cells were performed in the same manner as in Example 1.

### (1) Cryopreservation

After the culture in (4) of Example 1 finished, for each culture solution, the cells in each well were moved to a plastic tube having a volume of 15 mL. From the culture solution moved to the plastic tube, a portion having a volume of 10 µL was separated to another tube for counting the number of cells. The same amount of a trypan blue solution was added to 10 µL of the cell suspension, and the number of living cells was calculated.

A plastic tube containing the remaining cell culture solution was centrifuged (1,000 rpm, 5 minutes, room temperature) to remove the supernatant. Then, a cryopreservation solution was added to the precipitated cell pellets such that the cell density was 4 × 10⁶ cells/mL, thereby suspending the cells. As the cryopreservation solution, a cryopreservation solution A (90% FBS/10% DMSO) containing serum and DMSO, a cryopreservation solution B that does not contain serum but contains DMSO ("BAMBANKER", #CS-04-001, manufactured by NIPPON Genetics EUROPE GmbH), or a cryopreservation solution C ("Cell Reservoir One", #07579-24, manufactured by Nacalai Tesque Inc.) containing neither serum nor DMSO was used.

The prepared cell suspension was dispensed into cryotubes in 1 mL portions, and these tubes were placed in a cell cryopreservation container ("BICELL", manufactured by Nihon Freezer Co., Ltd.) and stored at -80°C.

### (2) Thawing and culture

After storage at -80°C, the cryotubes were immersed in a water bath at room temperature such that the cell suspension in the tubes was thawed. The entirety of the cell suspension in the tubes was moved to a plastic tube having a volume of 15 mL, the plastic tube was centrifuged (1,000 rpm, 5 minutes, room temperature) to remove the supernatant. Then, RPMI 1640/10% FBS/2-ME was added to the precipitated cell pellets such that the cell density was 4 × 10⁵ cells/mL and the cells were suspended, thereby preparing a cell suspension.

The prepared cell suspension was dispensed into T25 flasks in 5 mL portions. These T25 flasks were cultured (normal culture) for 8 days in an environment of 37°C, 5% CO₂, and 20% O₂.

### (3) FACS

The cells obtained after the culture in (2) described above were subjected to FACS analysis. The FACS analysis was performed in the same manner as in (6) of Example 1.

FIG. 4 is a dot plot showing the results of FACS on cells obtained by culturing cells that were cultured in a Th2 medium and then cryopreserved in the cryopreservation solution A in RPMI 1640/10% FBS/2-ME (differentiation-inducing factor deficient medium) in a normal environment. (A) is a plot in which the ordinate shows SSC and the abscissa shows FSC. (B) is a plot in which the ordinate shows the intensity of fluorescence emitted from the anti-CD62L antibody and the abscissa shows the intensity of fluorescence emitted from the anti-CD45RA antibody. (C) is a plot in which the ordinate shows the intensity of fluorescence emitted from the anti-CD127 antibody and the abscissa shows the intensity of fluorescence emitted from the anti-CD62L antibody.

In FIG. 4(A), the cells within the gated region are living cells. The cell viability after hypoxic culture was 27.1%, which was about the same as the cell viability in Example 2. On the other hand, the cell viability after normal culture was 2.3%, and there were surviving cells unlike in Example 2 in which substantially all cells died. Furthermore, it was confirmed that the surviving cell population was a cell population consisting of stem cell memory T cells, central memory T cells, and effector memory T cells just as the cell population cultured in a hypoxic environment (FIGS. 4(B) and 4(C)). Even though the cryopreservation solution B and the cryopreservation solution C were used for cryopreservation, a cell population consisting of stem cell memory T cells, central memory T cells, and effector memory T cells was obtained as well.

From these results, it was confirmed that in a case where naive CD4⁺ T cells were activated by differentiation induction, then frozen and thawed, and then cultured in a differentiation-inducing factor deficient medium in a normal environment, regardless of whether or not the cryopreservation solution used contains serum or DMSO, it was possible to convert the cells into memory T cells having acquired long-term viability just as in a case where the cells are cultured in a hypoxic environment.

### [Example 4]

Differentiation of naive CD8⁺ T cells recovered from peripheral blood collected from a 21-year-old human was induced, and then the cells were cryopreserved, thereby manufacturing memory lymphocytes. The recovery of the naive CD8⁺ T cells from human peripheral blood, the subsequent differentiation induction, and the removal of magnetic beads were performed in the same manner as (1) to (3) in Example 2.

### (1) Cryopreservation

From the cell suspension recovered into the FACS tube after the removal of the magnetic beads, a portion having a volume of 10 µL was separated to another tube for counting the number of cells. The same amount of a trypan blue solution was added to 10 µL of the cell suspension, and the number of living cells was calculated.

The entirety of the cell culture solution remaining in the FACS tube was moved to a plastic tube having a volume of 15 mL and then centrifuged (1,000 rpm, 5 minutes, room temperature) to remove the supernatant. Cryopreservation solutions were added to the cell pellets precipitated in the plastic tube such that the cell density was 4 × 10⁵ cells/mL, thereby suspending the cells. As the cryopreservation solutions, the three solutions used in Example 4 were used.

The prepared cell suspension was dispensed into cryotubes in 1 mL portions, and these tubes were placed in a cell cryopreservation container ("BICELL", manufactured by Nihon Freezer Co., Ltd.) and stored at -80°C.

### (2) Thawing and culture

After being stored at -80°C, the cells in the cryotubes were thawed and then cultured (normal culture) for 8 days in an environment of 37°C, 5% CO₂, and 20% O₂. The thawing and culture of the cells were carried out in the same manner as in (2) of Example 4.

### (3) FACS

The cells obtained after the culture in (2) described above were subjected to FACS analysis. The FACS analysis was performed in the same manner as in (6) of Example 1.

FIG. 5 is a dot plot showing the results of FACS on cells obtained by culturing cells that were cultured in a CD8-TA medium and then cryopreserved in the cryopreservation solution A in RPMI 1640/10% FBS/2-ME (differentiation-inducing factor deficient medium) in a normal environment. In FIG. 5(A), the cells within the gated region are living cells. The cell viability after hypoxic culture was 36.0%, which was about the same as the cell viability in Example 3. On the other hand, the cell viability after normal culture was 20.4%, and there were an extremely large number of surviving cells unlike in Example 3 in which substantially all cells died. The surviving cell population was a cell population which was mainly composed of stem cell memory T cells and central memory T cells and also contained effector memory T cells (FIGS. 5(B) and 5(C)) just as the cell population cultured in a hypoxic environment. Even though the cryopreservation solution B and the cryopreservation solution C were used for cryopreservation, a cell population mainly consisting of stem cell memory T cells and central memory T cells and also containing effector memory T cells was obtained as well.

From these results, it was confirmed that in a case where naive CD8⁺ T cells were activated by differentiation induction, then frozen and thawed, and then cultured in a differentiation-inducing factor deficient medium in a normal environment, regardless of whether or not the cryopreservation solution used contained serum or DMSO, it was possible to convert the cells into memory T cells having acquired long-term viability just as in a case where the cells are cultured in a hypoxic environment.

### [Example 5]

Differentiation of each of naive CD4⁺ T cells and naive CD8⁺ T cells recovered from human peripheral blood having been in cold storage was induced, and then the cells were cultured in a differentiation-inducing factor deficient medium in an environment with normal oxygen concentration, thereby manufacturing memory T lymphocytes.

### (1) Recovery of naive CD4⁺ T cells and induction of differentiation into CD4⁺ Th2 cells

A blood preparation was treated with a white blood cell removal filter before transfusion. Naive CD4⁺ T cells were recovered from the white blood cell removal filter that was used for filtering and then stored at 4°C for 3 days, and then differentiation into CD4⁺ Th2 cells was induced. The recovery of the naive CD4⁺ T cells from the white blood cell removal filter and differentiation induction were performed in the same manner as in (2) and (4) of Example 1.

### (2) Recovery of naive CD8⁺ T cells and induction of differentiation into CD8⁺ killer T cells

Naive CD8⁺ T cells were recovered from a white blood cell removal filter that was different from the white blood cell removal filter used in (1) described above and stored at 4°C for 3 days after use, and then differentiation into CD8⁺ killer T cells was induced. Magnetic beads were removed from the cells after differentiation induction. The recovery of the naive CD8⁺ T cells from the white blood cell removal filter, the subsequent differentiation induction, and the removal of magnetic beads were performed in the same manner as (1) to (3) in Example 2.

### (3) Culture in differentiation-inducing factor deficient medium

For each culture solution, the cells obtained after completion of differentiation induction were moved to a plastic tube having a volume of 15 mL. From the culture solution moved to the plastic tube, a portion having a volume of 10 µL was separated to another tube for counting the number of cells. The same amount of a trypan blue solution was added to 10 µL of the cell suspension, and the number of living cells was calculated.

A plastic tube containing the remaining cell culture solution was centrifuged (1,000 rpm, 5 minutes, room temperature) to remove the supernatant, and then RPMI 1640/10% FBS/2-ME was added to the precipitated cell pellets such that the cell density was 4 × 10⁵ cells/mL and the cells were suspended, thereby preparing a cell suspension.

The prepared cell suspension was dispensed into T25 flasks in 5 mL portions. These T25 flasks were cultured (normal culture) for 8 days in an environment of 37°C, 5% CO₂, and 20% O₂.

### (4) FACS

The cells obtained after the culture in (3) described above were subjected to FACS analysis. The FACS analysis was performed in the same manner as in (6) of Example 1.

FIG. 6(A) is a dot plot showing the results of FACS on cells obtained by culturing naive CD4⁺ T cells having been in cold storage in a Th2 medium and then culturing the cells for 8 days in RPMI 1640/10% FBS/2-ME (differentiation-inducing factor deficient medium) in a normal environment. FIG. 6(B) is a dot plot showing the results of FACS on cells obtained by culturing naive CD8⁺ T cells having been in cold storage in a CD8-TA medium and then culturing the cells for 8 days in RPMI 1640/10% FBS/2-ME (differentiation-inducing factor deficient medium) in a normal environment. In the drawings, the cells within the gated region are living cells.

In a case where the naive CD4⁺ T cells that had not been in cold storage were subjected to differentiation induction and then cultured in a normal environment, substantially all the cells died (Example 2). In contrast, in a case where the naive CD4⁺ T cells that had been in cold storage were subjected to differentiation induction and then cultured in a normal environment, the cell viability was as high as 12.1% (FIG. 6(A)). From the surface antigens, it was confirmed that the surviving cell population was a cell population consisting of stem cell memory T cells, central memory T cells, and effector memory T cells just as the cell population cultured in a hypoxic environment after differentiation induction.

Likewise, in a case where the naive CD8⁺ T cells that had not been in cold storage were subjected to differentiation induction and then cultured in a normal environment, substantially all the cells died (Example 3). In contrast, in a case where the naive CD8⁺ T cells that had been in cold storage were subjected to differentiation induction and cultured in a normal environment, the cell viability was 1.7%, and there were surviving cells (FIG. 6(B)). From the cell surface antigens, it was confirmed that the surviving cell population was a cell population which mainly consisted of stem cell memory T cells and central memory T cells and also contained effector memory T cells, just as the cell population cultured in a hypoxic environment after differentiation induction.

### [Example 6]

Differentiation of each of naive CD4⁺ T cells and naive CD8⁺ T cells recovered from human peripheral blood collected from a 59-year-old human was induced, and then the cells were cultured in a differentiation-inducing factor deficient medium, thereby manufacturing memory lymphocytes.

### (1) Collection of PBMC

PBMC were collected from peripheral blood in the same manner as in (1) of Example 1.

### (2) Recovery of naive CD4⁺ T cells and induction of differentiation into CD4⁺ Th2 cells

Naive CD4⁺ T cells were recovered from PBMC collected in (1) described above, and then differentiation into CD4⁺ Th2 cells was induced. The recovery of the naive CD4⁺ T cells from PBMC and the induction of differentiation into CD4⁺ Th2 cells were performed in the same manner as in (2) and (4) of Example 1.

### (3) Recovery of naive CD8⁺ T cells and induction of differentiation into CD8⁺ killer T cells

Naive CD8⁺ T cells were recovered from PBMC collected in (1) described above, and then differentiation into CD8⁺ killer T cells was induced. Magnetic beads were removed from the cells after differentiation induction. The recovery of the naive CD8⁺ T cells from PBMC, the subsequent differentiation induction, and the removal of magnetic beads were performed in the same manner as (1) to (3) in Example 2.

### (4) Culture in differentiation-inducing factor deficient medium

For each culture solution, the cells obtained after completion of differentiation induction were moved to a plastic tube having a volume of 15 mL. From the culture solution moved to the plastic tube, a portion having a volume of 10 µL was separated to another tube for counting the number of cells. The same amount of a trypan blue solution was added to 10 µL of the cell suspension, and the number of living cells was calculated.

A plastic tube containing the remaining cell culture solution was centrifuged (1,000 rpm, 5 minutes, room temperature) to remove the supernatant, and then RPMI 1640/10% FBS/2-ME was added to the precipitated cell pellets such that the cell density was 4 × 10⁵ cells/mL and the cells were suspended, thereby preparing a cell suspension.

The prepared cell suspension was dispensed into T25 flasks in 5 mL portions. These T25 flasks were cultured for 8 days in an environment of 37°C, 5% CO₂, and 4% or 20% O₂.

### (5) FACS

The cells obtained after the culture in (4) described above were subjected to FACS analysis. The FACS analysis was performed in the same manner as in (6) of Example 1.

FIGS. 7(A) and 7(C) are dot plots showing the results of FACS on cells obtained by culturing naive CD4⁺ T cells in a Th2 medium and then culturing the cells in RPMI 1640/10% FBS/2-ME (differentiation-inducing factor deficient medium). FIGS. 7(B) and 7(D) are dot plots showing the results of FACS on cells obtained by culturing naive CD4⁺ T cells in a Th2 medium and then culturing the cells in RPMI 1640/10% FBS/2-ME. (A) and (B) on the upper side are dot plots of cells cultured in RPMI 1640/10% FBS/2-ME in a hypoxic environment (4% O₂), and (C) and (D) on the lower side are dot plots of cells cultured in RPMI 1640/10% FBS/2-ME in a normal environment (20% O₂). In the drawings, the cells within the gated region are living cells.

In a case where the naive CD4⁺ T cells collected from a 20-year-old human were subjected to differentiation induction and then cultured in a normal environment, substantially all the cells died (Example 2). In contrast, the naive CD4⁺ T cells collected from a 59-year-old human had a cell viability which was 51.4% in a case where the cells were subjected to differentiation induction and then cultured in a normal environment and had an extremely high cell viability in a case where the cells were cultured in a hypoxic environment although the cell viability was less than 60.9% (FIGS. 7(A) and 7(C)). From the surface antigens, it was confirmed that the surviving cell population was a cell population consisting of stem cell memory T cells, central memory T cells, and effector memory T cells just as the cell population cultured in a hypoxic environment after differentiation induction.

Likewise, in a case where the naive CD8⁺ T cells collected from a 21-year-old human were subjected to differentiation induction and then cultured in a normal environment, substantially all the cells died (Example 3). In contrast, the naive CD8⁺ T cells collected from a 59-year-old human had a cell viability which was 19.0% in a case where the cells were subjected to differentiation induction and then cultured in a normal environment and had a sufficiently high cell viability in a case where the cells were cultured in a hypoxic environment although the cell viability was less than 24.9% (FIGS. 7(B) and 7(D)). From the cell surface antigens, it was confirmed that the surviving cell population was a cell population which mainly consisted of stem cell memory T cells and central memory T cells and also contained effector memory T cells, just as the cell population cultured in a hypoxic environment after differentiation induction.

### [Example 7]

Naive B cells recovered from human peripheral blood were subjected to differentiation induction and then cultured in a differentiation-inducing factor deficient medium in a hypoxic environment such that a cellular quiescence was induced, thereby manufacturing cells viable for a long period of time in a state of having ceased expansion without stimulation by a differentiation-inducing factor.

### (1) Recovery of naive B cells from PBMC

Naive B cells were recovered from a cell suspension prepared in the same manner as in (1) of Example 1. The naive B cells were recovered using a commercially available naive B cell isolation kit ("EasySep human naive B cell isolation kit", #17254, manufactured by STEMCELL Technologies.) according to the manual attached to the kit.

From the purified naive B cell suspension, a portion having a volume of 10 µL was separated to another tube for counting the number of cells. The same amount of a trypan blue solution was added to 10 µL of the cell suspension, and the number of living cells was calculated.

### (2) Induction of differentiation of naive B cells

First, the naive B cell suspension prepared and purified in (1) described above was moved to a plastic tube having a volume of 15 mL and then centrifuged (1,000 rpm, 5 minutes, room temperature) to remove the supernatant. The step 1 medium was added to the cell pellets precipitated in the plastic tube such that the cell density was 2 × 10⁵ cells/mL, thereby suspending the cells.

The obtained cell suspension was dispensed at 1 mL/well into a 24-well plate coated with a goat anti-human Ig mixed secondary antibody (#H17000, manufactured by Invitrogen). The plate was cultured for 5 days in an environment of 37°C, 5% CO₂, and 20% O₂.

After the culture finished, the culture solution in each well was recovered into a plastic tube having a volume of 15 mL and then centrifuged (1,000 rpm, 5 minutes, room temperature) to remove the supernatant. IMDM/10% FBS/2-ME (5 mL) was added to the cell pellets precipitated in the plastic tube to suspend the cells, and then the suspension was centrifuged again (1,000 rpm, 5 minutes, room temperature) to remove the supernatant. The step 2 medium was added to the cell pellets precipitated in the plastic tube such that the cell density was 4 × 10⁵ cells/mL, thereby suspending the cells.

The obtained cell suspension was dispensed at 1 mL/well to an uncoated 24-well plate. The plate was cultured for 4 days in an environment of 37°C, 5% CO₂, and 1% O₂ (hypoxic culture).

### (3) Culture in differentiation-inducing factor deficient medium

After the hypoxic culture in (2) described above finished, for each culture solution, the cells in each well were moved to a plastic tube having a volume of 15 mL. From the culture solution moved to the plastic tube, a portion having a volume of 10 µL was separated to another tube for counting the number of cells. The same amount of a trypan blue solution was added to 10 µL of the cell suspension, and the number of living cells was calculated.

A plastic tube containing the remaining cell culture solution was centrifuged (1,000 rpm, 5 minutes, room temperature) to remove the supernatant. Then, 1 mL of IMDM/10% FBS/2-ME was added to the precipitated cell pellets for 1 well such that the cells were suspended, thereby preparing a cell suspension.

The prepared cell suspension was dispensed at 1 mL/well to an uncoated 24-well plate. The plate was cultured for 6 days in an environment of 37°C, 5% CO₂, and 1% O₂. Control cells were cultured (normal culture) for 6 days in an environment of 37°C, 5% CO₂, and 20% O₂.

### (4) FACS

FACS analysis was performed on the cells obtained after the culture in (3) described above in the same manner as in (6) of Example 1, except that an antibody cocktail for B cells (5 µL of anti-CD20 antibodies, 5 µL of anti-CD27 antibodies, 5 µL of anti-CD38 antibodies, and 7 µL of 7-AAD) was used instead of the antibody cocktail for T cells.

The naive B cells, effector B cells (plasma cells), memory B cells, germinal center B cells, and transitional B cells each had the cell surface antigens shown in Table 2.

**[Table 2]**

| Cells | Surface antigen |
|---|---|
| Naive B cells | CD20+ / CD27- / CD38- |
| Plasma cells | CD20- / CD27+ / CD38+ |
| Memory B cells | CD20+ / CD27+ / CD38- |
| Germinal center B cells | CD20+ / CD27+ / CD38+ |
| Transitional B cells | CD20+ / CD27- / CD38+ |

FIG. 8 shows dot plots of a cell population of activated B cells (cells obtained after the completion of hypoxic culture in (2) described above) obtained by inducing differentiation of the naive B cells and a cell population obtained by culturing the activated B cells for 6 days in a differentiation-inducing factor deficient medium in a hypoxic environment (cells under no stimulation for 6 days). FIGS. 8(A) and 8(B) are plots in which the ordinate shows SSC and the abscissa shows FSC. FIG. 8(C) is a graph in which the intensity of fluorescence emitted from the anti-CD27 antibody is plotted on the ordinate and the intensity of fluorescence emitted from the anti-CD20 antibody is plotted on the abscissa. FIG. 8(D) is a graph in which the intensity of fluorescence emitted from the anti-CD38 antibody is plotted on the ordinate and the intensity of fluorescence emitted from the anti-CD27 antibody is plotted on the abscissa. In the plots of FIGS. 8(A) and 8(B), the cells within the gated region are living cells.

As a result, it was confirmed that the cell viability obtained after hypoxic culture in the differentiation-inducing factor deficient medium was 0.3%, and that most (92.4%) of the cells were plasma cells as shown in the result of surface antigens. Usually, plasma cells survive only for 2 or 3 days. However, in the present experiment, long living plasma cells were obtained which were able to expand for 6 days in a differentiation-inducing factor deficient medium.

### [Example 8]

Differentiation of naive B cells was induced using IL-4 and LPS, which is outer membrane components of Gram-negative bacteria. In this way, undifferentiated and quiescent B cells having acquired long-term viability independent of a differentiation-inducing factor were manufactured from the naive B cells.

### (1) Recovery of naive B cells from PBMC

A suspension of purified naive B cells was prepared from blood collected from a 21-year-old human in the same manner as in Example 7, and the number of living cells was calculated.

### (2) Induction of differentiation of naive B cells

First, the naive B cell suspension prepared and purified in (1) described above was moved to a plastic tube having a volume of 15 mL and then centrifuged (1,000 rpm, 5 minutes, room temperature) to remove the supernatant. An LPS/IL-4 medium was added to the cell pellets precipitated in the plastic tube such that the cell density was 1 × 10⁵ cells/mL, thereby suspending the cells.

The obtained cell suspension was dispensed at 1 mL/well to an uncoated 24-well plate. The plate was cultured for 7 days in an environment of 37°C, 5% CO₂, and 1% O₂ (hypoxic culture).

### (3) Culture in differentiation-inducing factor deficient medium

After the culture finished, the plate was centrifuged (1,000 rpm, 5 minutes, room temperature) to remove the supernatant from each well. Next, 1 mL of RPM1 1640/10% FBS/2-ME was added to each well to suspend the cells, and then the plate was cultured for 7 days in an environment of 37°C, 5% CO₂, and 1% O₂ (hypoxic culture).

### (4) FACS

FACS analysis was performed on the cells obtained after the culture in (3) described above, in the same manner as in (4) of Example 7.

FIG. 9 shows dot plots of a cell population of activated B cells (cells obtained after the completion of hypoxic culture in (2) described above) obtained by inducing differentiation of the naive B cells and a cell population obtained by culturing the activated B cells for 7 days in a differentiation-inducing factor deficient medium in a hypoxic environment (cells under no stimulation for 7 days). FIGS. 9(A) and 9(B) are plots in which the ordinate shows SSC and the abscissa shows FSC. FIG. 9(C) is a graph in which the intensity of fluorescence emitted from the anti-CD27 antibody is plotted on the ordinate and the intensity of fluorescence emitted from the anti-CD20 antibody is plotted on the abscissa. FIG. 9(D) is a graph in which the intensity of fluorescence emitted from the anti-CD38 antibody is plotted on the ordinate and the intensity of fluorescence emitted from the anti-CD27 antibody is plotted on the abscissa. In the plots of FIGS. 9(A) and 9(B), the cells within the gated region are living cells.

As a result, it was confirmed that the cell viability obtained after hypoxic culture in the differentiation-inducing factor deficient medium was 12.4%, and that most of the cells were long living transitional B cells as shown in the result of surface antigens.

In a case where the activation and culture of B cells in (2) described above were carried out in an LPS/IL-4 medium under conditions of 20% O₂, 75% of the cells were still alive until day 4 of culture. However, substantially all cells died on the day 7 of culture (viability: 0.6%).

### [Example 9]

Differentiation of naive B cells was induced using IL-2, IL-6, and ODN2006. In this way, undifferentiated and quiescent B cells having acquired long-term viability independent of a differentiation-inducing factor were manufactured from the naive B cells.

### (1) Recovery of naive B cells from PBMC

A suspension of purified naive B cells was prepared from blood collected from a 59-year-old human in the same manner as in Example 7, and the number of living cells was calculated.

### (2) Induction of differentiation of naive B cells

First, the naive B cell suspension prepared and purified in (1) described above was moved to a plastic tube having a volume of 15 mL and then centrifuged (1,000 rpm, 5 minutes, room temperature) to remove the supernatant. An ODN2006 medium was added to the cell pellets precipitated in the plastic tube such that the cell density was 1 × 10⁵ cells/mL, thereby suspending the cells.

The obtained cell suspension was dispensed at 1 mL/well into a 24-well plate coated with a goat anti-human IgD polyclonal antibodies (NBP2-50086, manufactured by Novus Biologicals, LLC.) The plate was cultured for 6 days in an environment of 37°C, 5% CO₂, and 1% O₂ (hypoxic culture).

### (3) Culture in differentiation-inducing factor deficient medium

After the culture finished, the plate was centrifuged (1,000 rpm, 5 minutes, room temperature) to remove the supernatant from each well. Next, 1 mL of RPMI 1640/10% FBS/2-ME was added to each well to suspend the cells, and then the plate was cultured for 8 days in an environment of 37°C, 5% CO₂, and 1% O₂ (hypoxic culture).

### (4) FACS

FACS analysis was performed on the cells obtained after the culture in (3) described above, in the same manner as in (4) of Example 7.

FIG. 10 shows dot plots of a cell population of activated B cells (cells obtained after the completion of hypoxic culture in (2) described above) obtained by inducing differentiation of the naive B cells and a cell population obtained by culturing the activated B cells for 8 days in a differentiation-inducing factor deficient medium in a hypoxic environment (cells under no stimulation for 8 days). FIGS. 10(A) and 10(B) are plots in which the ordinate shows SSC and the abscissa shows FSC. FIG. 10(C) is a graph in which the intensity of fluorescence emitted from the anti-CD27 antibody is plotted on the ordinate and the intensity of fluorescence emitted from the anti-CD20 antibody is plotted on the abscissa. FIG. 10(D) is a graph in which the intensity of fluorescence emitted from the anti-CD38 antibody is plotted on the ordinate and the intensity of fluorescence emitted from the anti-CD27 antibody is plotted on the abscissa. In the plots of FIGS. 10(A) and 10(B), the cells within the gated region are living cells.

As a result, it was confirmed that the cell viability after the hypoxic culture in a differentiation-inducing factor deficient medium was 0.2%, 55.1 % of the cells were long living germinal center B cells, and 34.4% of the cells were long living transitional B cells. The cell viability after the hypoxic culture in a differentiation-inducing factor deficient medium was approximately the same as the cell viability in Example 7, which indicates that naive T cells collected from a human aged 50 or over are more easily converted into memory cells compared to naive T cells collected from a human in his/her 20s, but aging does not exert a memory cell conversion-facilitating effect on B cells.

## Claims

1. A manufacturing method of memory T cells, comprising:
an activation step of activating naive T cells with a differentiation-inducing factor; and
a memory-formation step of culturing the activated T cells in the absence of a differentiation-inducing factor after the activation step to manufacture memory T cells,
wherein the activated T cells are cultured in a hypoxic environment in the memory-formation step.

2. The manufacturing method of memory T cells according to Claim 1,
wherein an oxygen concentration of the hypoxic environment is 0.5% to 5.0% by volume.

3. A manufacturing method of memory T cells, comprising:
an activation step of activating naive T cells with a differentiation-inducing factor; and
a memory-formation step of culturing the activated T cells in the absence of a differentiation-inducing factor after the activation step to manufacture memory T cells,
wherein the naive T cells are cells having been subjected to a stress treatment, or
a stress treatment is performed on the activated T cells before the memory-formation step.

4. The manufacturing method of memory T cells according to Claim 3,
wherein the naive T cells are cells having been kept at 0°C to 10°C for 1 hour or longer.

5. The manufacturing method of memory T cells according to Claim 3,
wherein the naive T cells are cells collected from a human aged 50 or over.

6. The manufacturing method of memory T cells according to Claim 3,
wherein the memory-formation step is performed after the activated T cells are frozen and thawed.

7. The manufacturing method of memory T cells according to any one of Claims 1 to 6,
wherein the naive T cells are naive CD4⁺ T cells or naive CD8⁺ T cells.

8. The manufacturing method of memory T cells according to Claim 7,
wherein the differentiation-inducing factor is a differentiation-inducing factor for differentiating naive CD4⁺ T cells into CD4⁺ Th1 cells, for differentiating naive CD4⁺ T cells into CD4⁺ Th2 cells, or for differentiating naive CD8⁺ T cells into killer T cells.

9. A memory T cell-containing composition,
wherein a total number of living cells of stem cell memory T cells, central memory T cells, and effector memory T cells is equal to or more than 20% of a total number of cells contained in the composition.

10. A pharmaceutical composition comprising:
the memory T cell-containing composition according to Claim 9 as an active ingredient.

11. A manufacturing method of cells viable for a long period of time independently of a differentiation-inducing factor, the manufacturing method comprising:
an activation step of activating naive B cells with a differentiation-inducing factor; and
a long-term viability acquisition step of culturing the activated B cells in the absence of a differentiation-inducing factor after the activation step to manufacture cells viable independently of a differentiation-inducing factor,
wherein the activated B cells are cultured in a hypoxic environment in the long-term viability acquisition step.

12. The manufacturing method of cells viable for a long period of time independently of a differentiation-inducing factor according to Claim 11,
wherein the cells viable for a long period of time independently of a differentiation-inducing factor are one or more kinds of cells selected from the group consisting of plasma cells, germinal center B cells, and transitional B cells.
